# EUROPEAN PATENT SPECIFICATION

(11) **EP 0 366 770 B1**
(45) Date of publication and mention of the grant of the patent: **09.02.1994**
(21) Application number: 89905671.7
(22) Date of filing: 12.05.1989
(51) Int. Cl.: A61K 9/50

(54) **LIPOSOMES COUPLED TO HORMONES**
AN HORMONE GEKOPPELTE LIPOSOME
LIPOSOMES ACCOUPLES A DES HORMONES

(30) Priority: 16.05.1988 US 194636
(43) Date of publication of application: 09.05.1990
(60) Divisional application: 93111532.3
(73) Proprietor: VESTAR, INC., San Dimas California 91773 (US)
(72) Inventor: KONIGSBERG, Paula, Jean, Pasadena, CA 91106 (US); RICHER, LeRoy, Leonard, San Gabriel, CA 91775 (US); SCHMIDT, Paul, Gardner, San Marino, CA 91108 (US); ULIANA, Joseph, Anthony, Sierra Madre, CA 91024 (US)
(74) Representative: Brown, David Leslie
(86) International application number: EP8900521
(87) International publication number: WO8911270

(56) References cited:
- WO-A-86/04232
- FR-A- 2 564 319
- GB-A- 2 157 172
- The Journal of Immunology, volume 138, no. 12, 15 June 1987, The American Association of Immunologists, (IS), O. Bakouche et al.: "Liposomes expressing IL 1 biological activity", pp. 4256-4262

## Description

The present invention relates to the field of drug delivery systems and more particularly to targeted drugs encapsulated in liposomes coupled to hormones e.g. cytokines.

Animal cells are characterized by the presence of receptor molecules on their membrane surface, which mediate a metabolic response of the cells upon binding to their specific ligands. In the human body, some of these membrane receptors are expressed by a restricted number of cell types and may even be specific for a given cell type. If the biological function of a particular cell type is prejudicial to the well-being of a patient it may be desirable to deliver therapeutic agents for blocking or lysing the unwanted cells. Specific targeting is commonly achieved by coupling therapeutic agents to a receptor-specific ligand.

In organ transplant surgery, it is necessary to suppress the immune system of the graft recipient to minimise the likelihood of graft rejection after surgery. Immunosupppressive agents that have been used successfully in clinical practice include Steroids, azathioprine and cyclosporin A. It has been also proposed to use antibodies to some particular T-cell membrane antigens either alone or conjugated to cytotoxic drugs. In a similar manner, immune system dysfunctions such as autoimmune diseases which are the direct consequence of inappropriate activity of T-cells may be also treated with immunosuppressive drugs.

In some other cases, the presence of a particular membrane receptor on a given cell type may also be correlated with a malignant condition of the cells. Indeed some membrane receptors known to be normally present in small amounts are expressed at a much higher density on tumour cells, which may therefore be removed by specific targeting of cytotoxic drugs.

However, many of the above treatments have undesirable side effects due to the fact that the cytotoxic effect usually extends beyond the particular cell type involved in allograft rejection or in causing the disorder. For example, an essential problem arising from the use of some known immunosuppressive drugs is that the immunosuppressive action makes the patient particularly susceptible to infection by bacteria or viruses that would be controlled by a normal immune system. Another problem more especially associated with the delivery of cytotoxic drugs as conjugates with antibodies is that the antibody may not be internalized into the cells. Thus, although the toxin-antibody conjugate exhibits specific binding, it fails to deliver the toxin to the unwanted cells. As a result of the low frequency of internalization, impractically high concentrations of toxin-antibody conjugates must be used with corresponding increased risk of overall toxicity.

Thus, there is still a need for providing a therapeutic agent which can block or lyse unwanted cells at a high level of specificity and efficacy. It has been now found that these requirements may be achieved by encapsulating a cytotoxic drug into liposomes and linking the drug-containing liposomes to hormones. By "hormone" is meant a protein or a peptide which has a cell receptor binding site and which is able to induce a metabolic response in the target cell upon binding to its receptor. This includes, e.g. endocrine hormones which are conveyed in vivo by the blood from one organ to another as well as paracrine hormones and cytokines which are cell-to-cell mediators.

General prior proposals to couple liposomes to hormones do exist in the art (see, for example, FR-A-2564319 and WO-A-86/04232). However, no specific teachings are provided as to how such coupling may be achieved with acceptably high efficiency.

The present invention is based on our surprising finding that, by immunologically protecting the bioactive site of a hormone prior to coupling the hormone to a liposome, efficient retention of hormone activity can be achieved, particularly if one or several amino acid residues which may be involved in the linkage (coupling) should be located in the region of the bioactive (receptor-binding) site of the hormone.

Chemical protection of bioactive sites of antibodies for coupling the same to liposomes has been previously described (see, for example, Jansons et al, Analytical Biochemistry 111 : 54-59 (1981)). However, such protection methods produced a rather low level of retained bioactivity and have not been widely adopted.

Accordingly, the present invention provides, as defined in the appended claims, the use of an immunological (antibody) blocking agent for reversibly protecting the biologically active cell receptor binding site of a hormone prior to coupling the hormone to a liposome.

The liposome/hormone conjugates so prepared may be used as delivery vehicles for targeted delivery of a diagnostic or pharmaceutically active substance, preferably a cytotoxic agent.

Preferably, the liposomes are coupled to cytokines, e.g. lymphokines and monokines such as interleukins 1 to 7, interferons α, β and λ, tumour necrosis factors α and β, T-cell replacing factor, macrophage inhibitory factor and colony stimulating factors e.g. G-CSF and GM-CSF. A preferred cytokine is IL-2. IL-2 is a naturally occurring mitogenic cytokine with a short half-life produced by T-cells within hours of stimulation by antigen. It induces the proliferation of activated T-cells upon binding to receptor molecules which are transiently expressed on the activated T-cell membrane as a result of the stimulation by the antigen.

Very effective protection of the binding site of the hormone is achieved by using a monoclonal antibody (MAb) specific for the binding site. After linkage of the hormones to the liposomes, or in any event after performing any reaction step that might otherwise result in interference with or blocking of the binding site, the antibody is removed from the binding site to yield a site capable of interacting with the appropriate cell receptors for the hormone.

More particularly, the binding site of IL-2 which is located between amino acid residues 20-30 of the molecule may be protected by a MAb which specifically recognizes this area. Such a MAb is commercially available from Genzyme Inc. under the reference DMS-1. Thus, for use in the present invention, the MAb is coupled to a stationary phase e.g., a resin or a gel. Then IL-2 is applied to the MAb containing phase and is chemically modified e.g., with succinimidyl-4-(p-maleimidophenyl)butyrate (SMPB) in a molar ratio of from 5:1 to 50:1. A linker-modified IL-2 which has been protected with a MAb specific to the binding site exhibits a substantially increased capacity to compete with native IL-2 for binding to the IL-2 receptors as compared to linker-modified IL-2 which has not been so protected. Thus, IL-2 coupled to SMPB after protection with a suitable MAb is approximately two-and-one-half times more effective in competing with ¹²⁵I-labelled IL-2 for murine HT2 T cell receptors than IL-2 coupled with SMPB without such protection.

Recombinant forms of human IL-2 are particularly preferred for use in the present invention, especially those which have been stabilized by mutation. For example, one or more amino acids may be deleted or replaced by other amino acids, to provide modified human IL-2 with a longer half-life, for example IL-2 in which the naturally occurring cysteine in position 125 has been replaced by serine or alanine. Alternatively, shortened forms of the molecule may provide useful ligands to be coupled to liposomes so long as the IL-2 fragment contains a functionally active binding site, i.e. the amino acid sequence comprised between residues 20-30 in the natural human IL-2.

It is known that there are two different IL-2 receptors designated the low- and high- affinity receptors. The low-affinity receptor is expressed both on resting and activated T-cells whereas the high-affinity receptor is present only on activated T-cells, in small amounts compared to the low-affinity receptor. Most of the biological effects of IL-2 are mediated by the high-affinity receptor since this receptor is the only one involved in the internalization of IL-2. Anti (IL-2 receptor) MAb's have been produced and coupled to cytotoxic drugs for possible therapeutic use e.g. for preventing or treating allograft rejection episodes which are the result of a proliferative burst of activated T-cells induced by IL-2. It was anticipated that anti (IL-2 receptor) MAb conjugates could be effective in killing activated T-cells without adversely affecting the resting T-cells which are constantly required for normal immune surveillance, e.g. for fighting infections. However, these MAb's (usually called anti-TAC MAb's) recognize only the low affinity receptor and thus fail to be internalized.

However, where the IL-2 molecule or an active fragment thereof is used for targeting, binding will be predominantly to the high-affinity receptor. Therefore, the advantages of drug-containing liposomes coupled to IL-2 over anti-TAC MAb conjugates are: internalization of the drug and highly specific targeting of activated T-cells.

Liposomes are closed spherical shell-like structures comprising a bilayer membrane enclosing an aqueous volume. The primary constituents of the bilayer membrane are amphipathic molecules which may include phospholipids of natural or synthetic origin. For use in the present invention, the liposomes comprise phospholipids in which the hydrophilic group is phosphatidylethanolamine. Preferably, they also comprise phospholipids in which the hydrophilic group is phosphatidylcholine. The hydrophobic groups associated with phosphatidylethanolamine or phosphatidylcholine may be a variety of saturated and unsaturated fatty acid moieties. Phospholipids dispersed in aqueous solution spontaneously form bilayers with the hydrocarbon tails directed inward and the polar headgroups outward to interact with water.

Most preferably, the liposomes for use in the present invention are partially or totally composed of distearoyl phosphatidylethanolamine (DSPE) and/or distearoyl phosphatidylcholine (DSPC) in various ratios. It is also preferred that a sterol such as cholesterol be present in the liposome membrane. Suitable weight ratios of lipid to sterol may be from 6:1 to 1:1.

Liposomes may be small unilamellar vesicles (SUV) as well as oligo- or multi-lamellar vesicles (MLV) in an onion-like form in which the concentric bilayer membranes are separated from each other by a layer of water. Preferably, liposomes for use in the present invention are SUVs having an external diameter of from about 30 to about 300 nanometers; most preferably SUVs have an external diameter of from 50 to 100 nanometers.

MLVs may be obtained by simple agitation of a mixture of phospholipids. Ultrasonic irradiation (sonication) of these structures leads to the formation of SUVs. MLVs and SUVs may be produced by a variety of different standard methods as reported, for example, in F. Szoka and D. Papahadopoulos, "Liposomes and their uses in biology and medicine" Ann. N.Y.Acad. Sci. 308, 1-482, (1978) in R.L. Juliano and D. Layton, "Liposomes as a drug delivery system" in Drug Delivery Systems p. 189-236, Oxford University Press, Inc., New York (1980) or in H.J. Paznanstey and R.L. Juliano, "Biological approaches to the controlled delivery of drugs: A critical review" in Pharmacological Review 36:277-336 (1984).

It will be appreciated by those skilled in the art that drug-containing liposomes may be simply prepared by adding the drug to the lipid mixture used for the preparation of liposomes as above described so that the drug be passively encapsulated.

By the term "liposomes' or "SUVs" is meant hereinafter empty liposomes or SUVs as well as drug-containing liposomes or SUVs.

Cytotoxic drugs for encapsulation into liposomes include any compound able to block or disturb the metabolic activity of cells, e.g. which adversely interfere with the replication of DNA or protein synthesis. Preferred cytotoxic drugs are mitomycin C, daunorubicin, doxorubicin, cis-platinium, 6-mercaptopurine, mephalan, actinomycin D, fluorodeoxyuridine, AZT, cyclosporin A, and methotrexate; the last being particularly preferred.

With respect to cyclosporin A, (Sandimmune®) intravenous administration of the pharmaceutical compound in treatment of organ transplantation or graft-versus-host disease is normally at a dosage of 3-4 mg/kg/day. Oral doses are higher by approximately a factor of three. When administered using the delivery vehicles of the present invention, lower dosages may be expected to achieve comparable effects in view of the targeted delivery of the drug to immune system cells responsible for the disease conditions.

Methotrexate has been successfully encapsulated in amounts of 20-200 µg/mg lipid the concentration of which was then adjusted to produce 0.3mM methotrexate in aqueous liposome dispersion for administration.

The actual process for linking liposomes to a hormone, having a biologically active cell receptor binding site, comprises:
(a) fixing a coupling agent to liposomes and then reacting with the hormone, or
(b) fixing a coupling agent to the hormone and reacting with liposomes or,
(c) fixing a first coupling agent to liposomes, fixing a second coupling agent to the hormone and then reacting the coupling moieties together,
the alternative process c) being particularly preferred.

It may also be desirable to introduce a linker arm having 4 to 6 carbon atoms between the liposome and the ligand so that steric hindrance may be avoided. This linker arm may be attached to the liposome or the hormone, preferably to the liposome. In this case, the use of a single coupling agent is preferred for linking the linker arm, as fixed to the liposome or to the hormone, to the remaining constituent.

Coupling agent-bearing liposomes may be obtained by covalently attaching the coupling agent to phosphatidylethanolamine, preferably distearoyl phosphatidylethanolamine. The modified phospholipid is then used alone or in combination with other lipids and/or sterols for producing liposomes as above described.

Coupling agents for covalent attachment to phospholipids are well-known in the art. Advantageously they are heterobifunctional agents such as succinimidyl-4-(p-maleimidophenyl)-butyrate (SMPB) or sulphur-containing succinimidyl compounds such as succinimidyl-S-acetylthioacetate (SATA) or N-hydroxysuccinimidyl-3-(2-pyridyldithio)-propionate (SPDP). Sulphur-containing succinimidyl compounds are preferred. SATA is particularly preferred.

Coupling agents may also be fixed to a hormone by covalent linkage involving a functional group of the amino acid residues of the hormone. Advantageously, the binding occurs at a residue on the hormone which is selected from the group consisting of lysine, cysteine, histidine and arginine residues. The lysine residue is particularly preferred.

Suitable coupling agents to be bound to hormones are heterobifunctional agents. Advantageously, they are selected from succinimidyl-S-acetylthioacetate (SATA), succinimidyl-4-(p-maleimidophenyl)butyrate (SMPB), sulpho-SMPB, N-(4-carboxy-cyclohexyl-methyl) maleimide (SMCC), sulpho-SMCC, M-maleimidobenzoyl-N-hydroxysuccinimide ester (MBS), sulpho-MBS, N-succinimidyl (4-iodoacetyl) aminobenzoate (SIAB) and sulpho-SIAB. The most preferred coupling agent is SMPB.

In one preferred embodiment, succinimidyl-S-acetylthioacetate-phosphatidylethanolamine is used to produce activated liposomes including SATA in the membrane which are then covalently bound to a SMPB-hormone conjugate, most preferably to SMPB-IL-2 conjugate.

### Brief description of the drawings

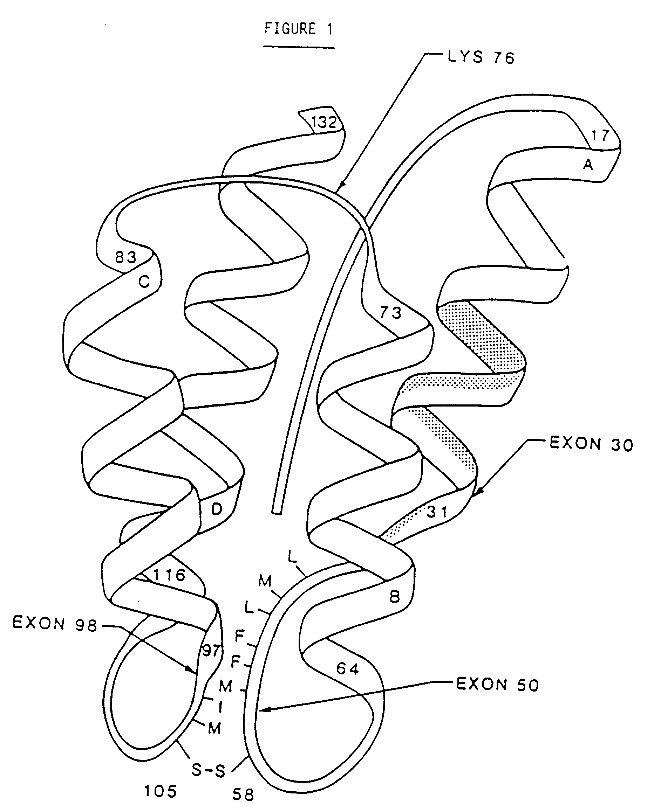

Figure 1 is a diagrammatic representation of the IL-2 molecule.

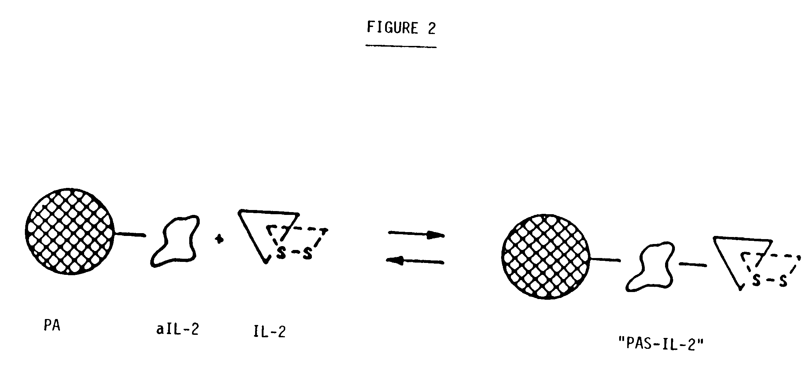

Figure 2 shows a method for protecting the receptor binding site of IL-2 in which PA refers to Protein A agarose, aIL-2 refers to the antibody to the receptor binding site of IL-2 and PAS IL-2 is the IL-2 bound to the resin.
Figure 3 shows a method for modifying IL-2 with SMPB using IL-2 in the form of PAS IL-2.
Figure 4 shows the reaction of phosphatidylethanolamine with SATA.
Figure 5 shows a method for generating a covalent bond between SMPB-modified IL-2 and SATA-modified liposomes.
Figure 6 shows a method for obtaining activated phosphatidylethanolamine in which the coupling moiety incorporates a 4-6 carbon linker arm.
Figure 7 shows a process for partially protecting the εNH₂ group of lysine residues with citraconic succinic anhydride before coupling to SATA.
Figure 8 shows a reaction for coupling phosphatidylethanolamine to camphoquinone-10-sulphonic acid.

### Example 1: Protection and modification of IL-2

A protein-A agarose resin is reacted with a MAb specific for the receptor binding site of IL-2 (available from Genzyme Inc; catalog reference DMS-1) by washing the resin with PBS buffer, applying the MAb to the resin, crosslinking the MAb to the resin in the presence of 12.5% glutaraldehyde in PBS for 60 min at 4°C and finally washing the resin again with buffer.

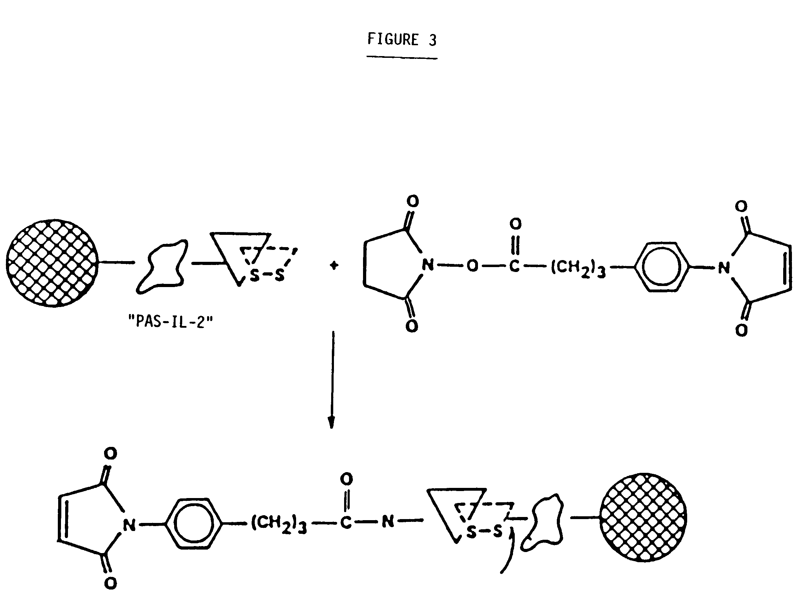

Then 50 µg of IL-2 in 100 µl buffer is applied to the resin coupled to the MAb, and modified with SMPB as indicated in Figure 3. SMPB is added to IL-2 in the molar ratio of 25:1. SMPB specifically reacts with the εNH₂ groups of lysine residues. The modified IL-2 is then eluted from the resin with 2M NaSCN in 0.05M Tris, pH 8 followed by a second elution treatment with 6% betaine in 0,2N acetic acid, all at 4°C.

An IL-2 receptor binding assay shows that the resulting SMPB is able to bind to the IL-2 receptor of target cells. Furthermore, in an ELISA assay, SMPB modified IL-2 is recognized by the monoclonal antibody to the receptor binding domain of IL-2, as is unmodified IL-2 eluted from the column under the same conditions.

In an alternative procedure, MBS (maleimidobenzoyl-N-hydroxy succinimide ester, a derivative of SMPB) is also used to modify IL-2. Use of this reagent also yields biologically active IL-2 when assayed in a receptor binding assay and in the anti-IL-2 ELISA.

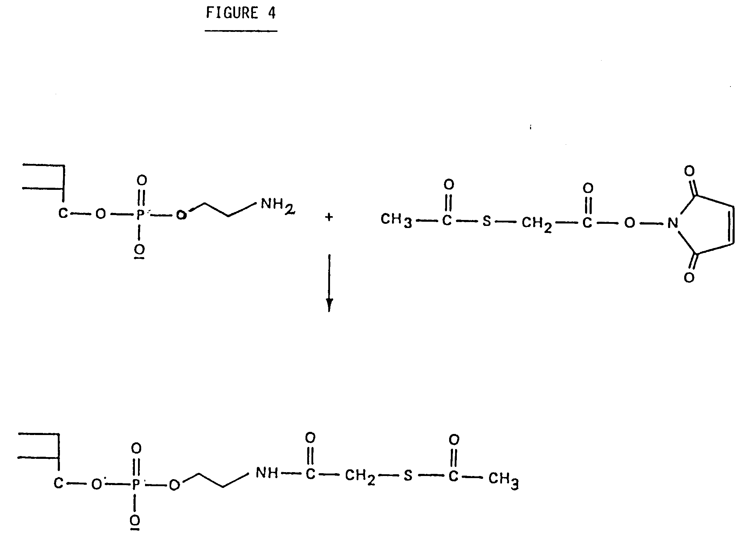

### Example 2: Synthesis of SATA-DSPE (See Fig. 4)

125 mg of fresh succinimidyl-s-acetylthioacetate (SATA) is mixed with 75 mg of distearoyl phosphatidylethanolamine (DSPE) in a round bottom flask. Then 15 ml CHCl₃:MeOH (1:1) is added, followed by 100-135 µl triethylamine. The flask is then flushed with nitrogen and the reaction carried out for two hours at room temperature whilst stirring.

The progress of the reaction is monitored by thin layer chromatography in CHCl₃:MeOH (7:3). The absence of a ninhydrin positive spot and the presence of a phosphomolybdic acid positive spot having an Rf of 0.57 indicates the presence of SATA-DSPE. SATA-DSPE may also be partially oxidized to the disulphide form SATA-DSPE-SATA-DSPE which has an Rf of about 0.37.

The material obtained is then evaporated to dryness and resuspended in 1 ml CHCl₃:MeOH (1:1). After addition of 15 ml acetonitrile, the mixture is held at -20°C for about 60 min to selectively precipitate SATA-DSPE, the unreacted DSPE remaining in solution. The precipitate is collected by filtration on a sintered glass filter and washed with acetonitrile. The washed SATA-DSPE is again dissolved by addition of CHCl₃:MeOH (1:1), collected in a second filter flask, transferred to another flask and evaporated.

During the above described procedure all steps are conducted as quickly as possible to minimize oxidation. However, small quantities of oxidized SATA-DSPE in the preparation are removed by preparative thin layer chromatography on a Kieselgel 60 plate (EM Science). Aluminium backed silica gel (no fluorescent indicator) plates are used with CHCl₃:MeOH (7:3) as the mobile phase. SATA-DSPE is located by iodine staining. The strips containing SATA-DSPE are cut out and extracted over a 30 minute period in CHCl₃:MeOH (1:1). The SATA-DSPE is concentrated to dryness, dissolved in CHCl₃:MeOH (1:1) and dispensed into vials. Each vial is flushed with nitrogen and stored at -20°C.

### Example 3 a): Preparation of empty liposomes.

Liposomes having the composition DSPC:cholesterol:SATA-DSPE in the molar ratio of 1.5:1:0.5 are prepared as follows:
10 mg of mixture of DSPC, cholesterol and SATA-DSPE in the above ratio is dissolved in 1 ml CHCl₃, dried to a film under nitrogen and then further dried under vacuum overnight.

The lipid film is dispersed in 1 ml PBS buffer pH 8.3,left at 65°C for 60 min, sonicated with a probe sonicator for 6 min at 65°C and held at the same temperature to form SUVs. The SUVs are then centrifuged at 13,000 xg for 10 min to remove any particles of titanium from the sonicator probe.

0,9 ml of the SUV preparation (about 10 mg of lipids) is then applied to a Sephacryl S300 column to remove the free SATA-DSPE (not incorporated into liposomes). The column is first presaturated with DSPC:cholesterol (2:1) SUVs in PBS, 1 mM EDTA 0,2 mM PMSF to maximise the recovery of SATA-DSPE SUVs in the column flow through. SATA-DSPE SUVs are recovered in a volume of 2.4 ml containing approximately 3,3 mg/ml of lipids. SUVs produced in this manner have a diameter of about 50 nm.

### Example 3 b): Preparation of drug-containing liposomes

Example 3 a) is repeated except that the PBS buffer used to disperse the lipid film contains 55 mg/ml of methotrexate. SATA-DSPE SUV's containing methotrexate are obtained.

### Example 4: Attachment of IL-2 to liposomes

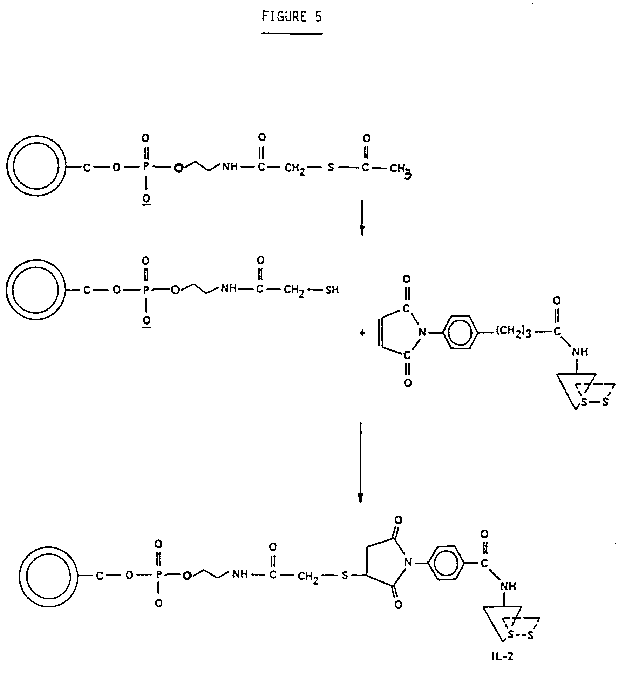

The modified SMPB-IL-2 of Example 1 is covalently coupled through its maleimido groups to the SUVs of Example 3 a) or 3 b).. With reference to Figure 5, the coupling is achieved as follows:
The SUVs containing SATA-DSPE are first activated by deacetylating the SATA residue so that it can react with SMPB-IL-2. 10 µl of freshly prepared 0,5M hydroxylamine is added to 1 ml of the SUV preparation of Example 1, (10 mg/ml in PBS). Incubation is performed for 30 min at 22°C under argon.

Then the pH of the SMPB-IL-2 preparation of Example 1 is adjusted to pH 6.5-7. A sample is then added to the deacetylated SUVs In the amount of 10-20 µg SMPB-IL-2 for 12 to 24 µg lipid. Incubation is performed for 2 hrs at 22°C under argon. Then to block any unreacted -SH groups, NEM is added to give a final concentration of 20 mM.

The pH of the vesicle-IL-2 suspension is adjusted to pH 9. A CM-Sepharose column (50 mM Tris, pH 9.0) was drained. The liposomes are applied to the column, the resin stirred, incubated 10 min, and again drained. Aliquots (0.5 ml) of buffer are added and fractions collected. All turbid or opalescent fractions are pooled and assayed.

The recovered SUV preparation contains SUVs which have 6 to 5040 molecules of bound IL-2 per SUV (50 nm diameter).

When Example 4 is repeated with the methotrexate-containing liposomes of Example 3 b) liposomes are obtained which have IL-2 bound to the outer surface and which contain methotrexate.

### Example 5: Synthesis of an extended linker arm to alleviate steric hindrance

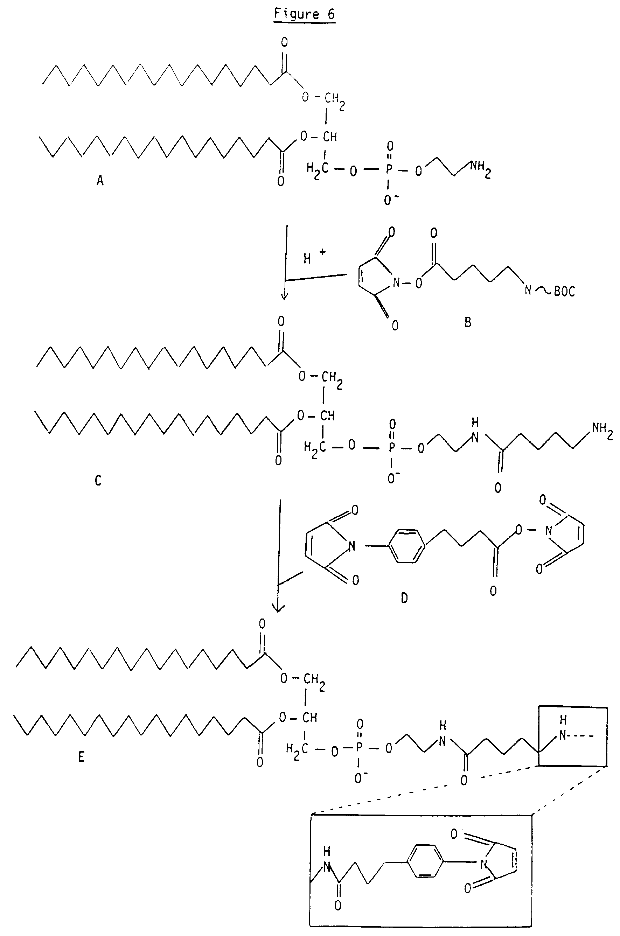

A preferred route to the synthesis (Figure 6) of generally useful activated lipids is as follows:
To a solution of distearoyl phosphatidylethanolamine (A) and succinimidyl-gamma-BOC-aminopentanoate (B) in chloroform-methanol, 9:1, is added a slight excess of triethylamine. The solvent is evaporated and the BOC group removed with a solution of trifluoroacetic acid in dichloromethane. The reaction of SMPB (D) with the distearoyl phosphatidylethyl-gamma-amino-pentanamide (C), left as residue after the trifluoroacetic acid reaction, is performed using the same conditions as for the first coupling reaction. The distearoyl phosphatidylethyl-4-(p-maleimidophenyl-butyramide) pentanamide (E) is purified using silica gel chromatography.

After purification, the activated lipid is incorporated into liposomes whereby drug or marker is entrapped in standard fashion. The liposomes are then ready for reaction with free protein sulfhydryls either present on the unmodified protein, or with derivatives prepared as described below.

For example, SATA may be used in limiting concentration. Thus, to a solution of IL-2 at about pH 8 may be added a solution of SATA in DMF or dioxane. The amount of substitution may be controlled by limiting the quantity of reagent. The sulfhydryl is then deblocked by the addition of hydroxylamine and the IL-2 immediately coupled to liposomes having the maleimido group on the surface.

### Example 6: Modification of IL-2 at selected (limited) lysine residues

The most abundant and available protein reaction sites are lysine εNH₂ groups. Since some of the lysine residues are located at the receptor binding site, any attempt to use the lysine residues for coupling must comprise limiting the lysine residues which actually undergo a reaction.

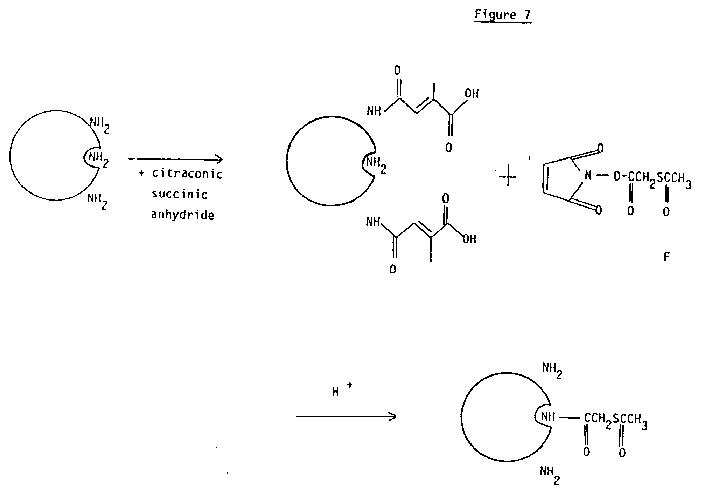

If the reactivity of the critical lysine residues is much faster than or at least comparable to most of the other lysine residues, the method shown in Figure 7 may be employed. In this method essentially all but a few lysine residues are first derivatized with a reversible blocking agent. Using only enough reagent so that the most reactive or accessible lysines are blocked, or after blocking followed by partial deblocking, the remaining lysine residues are then derivatized by using a large excess of a coupling agent, e.g. SATA. To a solution of IL-2 in borate buffer at pH 8.8 is added citraconic anhydride, maintaining the pH by addition of 1 N sodium hydroxide. The citraconic acid groups can be removed at pH 2, and this reaction can be interrupted by again raising the pH. SATA is added in excess to the solution at pH 8.8, derivatizing all free amines. The excess reagent is removed by dialysis and the remaining citraconic acid groups removed at pH 2. The solution is brought to neutral pH and sulfhydryl groups are formed by addition of hydroxylamine. SMPB liposomes are introduced and coupled to the protein.

### Example 7: Modification of IL-2 at arginine residues

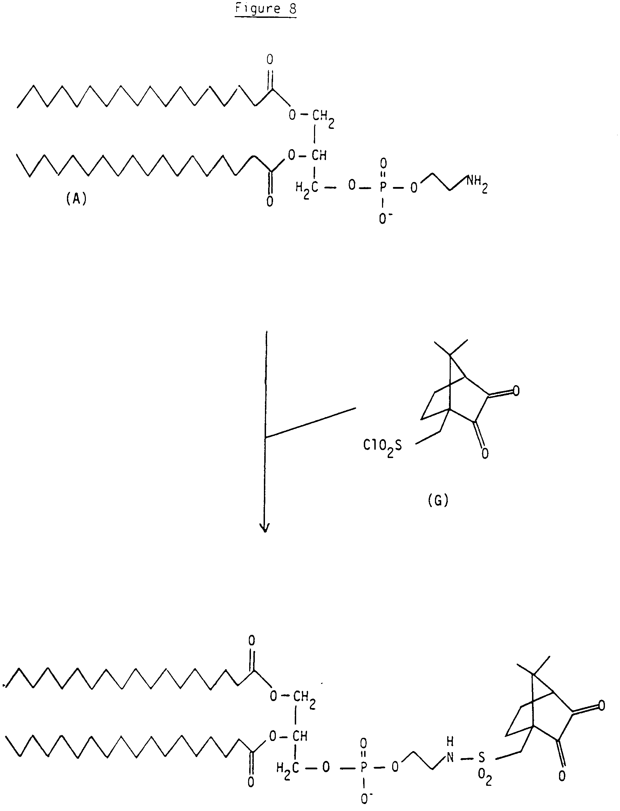

There are four arginines in interleukin-2, only one of which appears to be in a region that might affect binding. Thus, liposomes may be linked to IL-2 using an arginine specific reagent such as camphoquinone-10-sulphonic acid, as shown in Figure 8.

The camphorsulphonyl chloride, prepared using thionyl chloride, is added to phosphatidylethanolamine in the presence of pyridine. The camphorsulphonyl-phosphatidylethanolamine PE is then incorporated into liposomes and is reacted with IL-2 in borate buffer at pH 8.8. Vesicle-linked IL-2 is separated from unreacted protein on a Sephadex G-150 column.

### Example 8: Modification at histidine sites on the ligand

Histidine may be chemically modified via reagents such as bromoacetamide compounds. His-70 of IL-2 in particular, does not affect binding activity. A heterobifunctional reagent based on bromoacetamide may be synthesized to give an attachment point for the liposome. Specifically, this is: 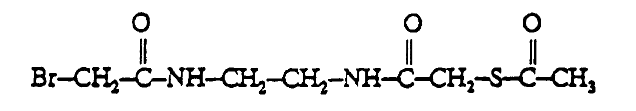
This compound is prepared by addition of bromoacetylbromide to ethylene diamine (1:1) followed by reaction of the remaining amine with SATA. Reaction with IL-2 histidine in 1M acetate buffer pH 5.5 produces, at the histidine imidazole group, the 3'-N-substituted SATA derivative. Addition of hydroxylamine deacetylates the SATA label to give a free sulfhydryl group available for reaction with SMPB liposomes.

### Example 9: Assays for monitoring receptor binding activity of the liposome/IL-2

Anti-proliferation measurements were performed by growing cells possessing the high affinity interleukin-2 receptor for several days in:
a. culture media in the absence of any form of interleukin-2,
b. culture media supplemented with an effective dose of free cytostatic agent,
c. culture media supplemented with a dose of the liposome/IL-2 complex of Example 4, and
d. culture media supplemented with a dose of liposome/IL-2 complex of Example 4 containing a cytostatic agent.

After an appropriate treatment period determined in previous studies, the cells are pulse-labelled with ³H-thymidine. Following a 3-4 hour labelling period, all cultures are then harvested on glass-fiber filter strips and ³H-thymidine incorporation measured as an index of cellular proliferation. The effectiveness of the liposome/IL-2 containing cytostatic agent complex is thus determined by comparison to cells cultured in the absence of any form of the drug. In vivo biodistribution of the liposome/IL-2 complexes may be ascertained using known techniques, which are described in Example 10.

### Example 10: Biodistribution studies

A preferred procedure for measuring biodistribution employs the radionuclide Indium-111. The liposome-encapsulated Indium will be in the form of one or two chelate complexes. For biodistribution studies measuring the in vivo fate of the liposome/IL-2 formulations, the chelating agent ethylenediamine tetraacetic acid (EDTA) is preferably used; for total in vivo uptake by target cells nitrilotriacetic acid (NTA) is preferably used.

In a conventional biodistribution study, SUVs of a given composition are prepared in the presence of chelating agent and Indium-111 for passive encapsulation of indium, or the ionophore A23187 for subsequent active encapsulation of Indium-111 into preformed liposomes. In either situation the liposomes are separated from unencapsulated materials by treatment with additional EDTA, followed by column chromatography.

Biodistribution measurements are conducted by injecting the study animals intravenously with the ¹¹¹In-liposome/IL-2 complex. At several times after injection (for example at 1, 3, 6 and 24 hours), animals are sacrificed and tissues collected for gamma counting. Liposomes loaded with ¹¹¹In-EDTA may be used for initial biodistribution studies. This tightly bound chelate complex allows measurement of in vivo retention times for the liposomes. Experiments performed relating to the present invention have shown that ¹¹¹In-EDTA alone is rapidly cleared in vivo. Therefore, any recovered Indium-111 activity may be interpreted as ¹¹¹In-liposome/IL-2.

Uptake of liposome/IL-2 by lymphocytes in a test animal may be studied using the ¹¹¹In-NTA complex. This relatively weak chelate complex has been shown to maintain Indium-111 in the aqueous compartment of liposomes, but quickly to release Indium-111 once the chelate is in the proximity of protein or carbohydrate macromolecules. Therefore, treatment of study animals with ¹¹¹In-NTA-liposome/IL-2 formulations provides a means for measuring cumulative uptake by lymphocytes.

This may be accomplished by treating animals with the ¹¹¹In-NTA formulations. Test animals may be given single or multiple (2-3) injections of the ¹¹¹In-NTA liposomes by either intravenous or intraperitoneal routes of administration. At various times after treatment, animals are sacrificed and lymphocytes isolated from, for example, blood, spleen, and peritoneum by centrifugation on Ficoll gradients. The isolated lymphocytes are then counted for Indium-111 in a gamma counter. Biodistribution studies in healthy rats have shown that the ¹¹¹In-liposome/IL-2 complex of Example 4 has extended circulation half-life, and does not attach to macrophages or unactivated T cells.

In vivo biodistribution studies, in addition to directly measuring liposome interaction with T-lymphocytes, may be used to evaluate the extent of up take of the liposome/IL-2 formulations by the reticulo-endothelial system (RES), particularly at liver, spleen and lymph node sites. It is widely known that many types of phospholipid vesicles are rapidly taken up in the liver and spleen, thus removing them from blood. Large liposomes and vesicles with various carbohydrate and protein groups attached are two particularly susceptible types. Therefore, the nature of the RES uptake is of importance for the practice of the present invention. Suitable doses may be established from such a test using standard procedure.

In order to avoid RES uptake of a non-specific nature and involving primarily macrophages_{,} to the extent this presents a hindrance to liposome/lymphocyte interaction for a given liposome/ interleukin-2 formulation, RES blockage by Prior treatment with liposomes directed to RES sites may be employed. Alternatively, additional modification to the surface of liposome/interleukin-2 formulations_{,} using for example a process similar to opsonization to enhance liposome interaction with blood lymphocytes by prolonging circulation times may be employed. Still further, RES uptake can be minimized by local administration of the interleukin-2 liposome preparation as a depot at the critical site, such as the site of a graft or an organ that is undergoing autoimmune attack.

### Example 11: Activated T-cell specificity

Efficacy of liposome/IL-2 preparations may be demonstrated using animal models in which T-lymphocytes can be reproducibly activated. Two models that are readily available are the rat skin allograft model and the rat burn model (30% body surface). Using either model, groups of animals would receive either liposome/IL-2-containing the immunosuppressive drug, or only liposome/IL-2. Retention of the allograft as the animal undergoes a protocol of liposome/IL-2 treatment is then measured. Those receiving liposome/IL-2 without the liposome-incorporated drug will readily reject the graft due to presence and cytolytic activity of activated T-cells in the area of the graft. Alternatively, liposome/IL-2 containing a drug of choice may also be used in the course of either the rat or mouse leukemia model. Tests have shown that the liposome/IL-2 complex of Example 4 is attracted to activated T cells in vitro.

The present delivery vehicles may be used in a variety of therapeutic contexts, including mammalian disease therapy and in vivo diagnosis, and biodistribution studies have shown significant advantages. When practiced using small unilamellar liposomes, which have been shown both to be useful in targeting solid tumors and to have greater circulation times than other vehicles, the present delivery vehicles may be used to deliver medicinal agents or diagnostic markers (as for example radioactive labels, fluorescent molecules and NMR-imaging agents such as magnetite) to neoplastic cells or particular organs of the body such as the liver. As contemplated herein, where the cell population of interest is characterized by a cellular receptor such as the interleukin-2 receptor, the present delivery vehicles may be utilized to deliver cytotoxic, regulatory, diagnostic or other molecules in a targeted manner. Thus, in the case of the liposome/IL-2 delivery vehicle of the present invention, active agents may be delivered to lymphocytes so as to treat, regulate or diagnose conditions involving malfunction of the immune system (including genetic or non-genetic autoimmune diseases such as rheumatoid arthritis, juvenile onset diabetes, systemic lupus erythematosus and others, as well as transplantation responses such as graft-versus-host disease), lymphocyte-related cancers (including lymphomas and leukemias such as adult or chronic myelogenous leukemias and hairy-cell leukemia) and helper T-cell disorders (including viral disorders such as those associated with HIV-infected T-cells).

## Claims

1. Liposomes which are covalently coupled to a hormone having a cell receptor binding site which is reversibly protected by an antibody specific for the binding site.

2. Liposomes according to claim 1 in which the antibody is a monoclonal antibody.

3. Liposomes according to claim 1 or claim 2 in which the hormone is a cytokine hormone.

4. Liposomes according to claim 3 in which the cytokine hormone is an interleukin 1 to 7.

5. Liposomes according to claim 4 in which the cytokine hormone is interleukin 2 (IL-2).

6. Liposomes according to any one of claims 1 to 5 which encapsulate at least one cytotoxic drug.

7. Liposomes according to any one of claims 1 to 6 the membrane of which comprises phosphatidylethanolamine.

8. Liposomes according to any one of claims 1 to 7 which are coupled to the hormone by a coupling moiety comprising a SATA and a SMPB residue said SATA residue being linked to the liposomes and said SMPB residue being linked to the hormone.

9. Liposomes according to any one of claims 1 to 7 which are coupled to the hormone by a coupling moiety comprising a 4 to 6 carbon linker arm.

10. Liposomes according to any one of claims 1 to 9 which are small unilamellar vesicles.

11. Liposomes according to any one of claims 1 to 10 which are coupled to the hormone at an amino acid residue of the hormone which is not located at the receptor binding site and which is selected from lysine, cysteine, arginine and histidine residues.

12. Liposomes according to claim 11 in which the coupling takes place at a lysine residue of the hormone.

13. A method for preparing liposomes which are covalently coupled to a hormone having a biologically active cell receptor binding site, comprising deprotecting the protected cell receptor binding site of the hormone coupled to the liposomes of any one of claims 1 to 12.

14. A process for coupling liposomes to a hormone having a biologically active cell receptor binding site, the coupling taking place at a site of the hormone which is not the said biologically active binding site, in which the said binding site is reversibly protected, prior to coupling, by an antibody specific for the said binding site, and is deprotected after performing any reaction step that might otherwise result in interference with, or blocking of, the said binding site.

15. A process according to claim 14 in which deprotection takes place after linkage of the hormone to the liposomes.

16. A process according to claim 14 or claim 15 in which the antibody is a monoclonal antibody.

17. A process according to any one of claims 14 to 16 in which the hormone is a cytokine hormone.

18. A process according to claim 17 in which the cytokine hormone is an interleukin 1 to 7.

19. A process according to claim 18 in which the cytokine hormone is interleukin 2 (IL-2).

20. A process according to any one of claims 14 to 19 in which the liposomes encapsulate at least one cytotoxic drug.

21. A process according to any one of claims 14 to 20 in which the liposome membrane comprises phosphatidylethanolamine.

22. A process according to any one of claims 14 to 21 which comprises fixing to the liposomes a coupling moiety which is the coupling residue of a coupling agent selected from succinimidyl-4-(P-maleimidophenyl)butyrate (SMPB), and sulphur-containing succinimidyl compounds.

23. A process according to claim 22 which comprises fixing to liposomes a coupling moiety which is the coupling residue of succinimidyl-S-acetylthioacetate (SATA).

24. A process according to any one of claims 14 to 23 which comprises fixing to the hormone a coupling moiety which is the coupling residue of a coupling agent selected from succinimidyl-4-(p-maleimidophenyl)butyrate (SMPB), sulpho-SMPB, N-(4-carboxy-cyclohexyl-methyl) maleimide (SMCC), sulpho-SMCC, N-maleimidobenzoyl-N-hydroxysuccinimide ester (MBS), sulpho-MBS, N-succinimidyl-(4-iodoacetyl)-aminobenzoate (SIAB) and sulpho-SIAB.

25. A process according to claim 24 which comprises fixing a coupling moiety which is a coupling residue of succinimidyl-4-(p-maleimidophenyl)-butyrate (SMPB) to the hormone.

26. A process according to any one of claims 14 to 25 which comprises fixing a coupling moiety which is a coupling residue of succinimidyl-S-acetylthioacetate (SATA) to liposomes, fixing a coupling moiety which is a coupling residue of SMPB to the hormone and then reacting the coupling moieties together.

27. A process according to any one of claims 14 to 26 in which the cell receptor binding site of the hormone is reversibly protected before fixing the coupling moiety.

28. A process according to any one of claims 14 to 27 in which the liposomes are small unilamellar vesicles.

29. A process according to any one of claims 14 to 28 in which the liposomes are coupled to the hormone at an amino acid residue on the hormone which is not located at the receptor binding site and which is selected from lysine, cysteine, arginine and histidine residues.

30. A process according to claim 29 in which the coupling takes place at a lysine residue of the hormone.

## Patentansprüche

1. Liposome, die kovalent an ein Hormon mit einer Zellrezeptorbindungsstelle gekoppelt sind, die reversibel durch einen für die Bindungsstelle spezifischen Antikörper geschützt ist.

2. Liposome nach Anspruch 1, bei denen der Antikörper ein monoklonaler Antikörper ist.

3. Liposome nach Anspruch 1 oder Anspruch 2, bei denen das Hormon ein zytokines Hormon ist.

4. Liposome nach Anspruch 3, bei denen das zytokine Hormon ein Interleukin 1 bis 7 ist.

5. Liposome nach Anspruch 4, bei denen das zytokine Hormon Interleukin 2 (IL-2) ist.

6. Liposome nach einem der Ansprüche 1 bis 5, die zumindest einen cytotoxischen Wirkstoff einschließen.

7. Liposome nach einem der Ansprüche 1 bis 6, deren Membran Phosphatidylethanolamin enthält.

8. Liposome nach einem der Ansprüche 1 bis 7, die an das Hormon durch ein Kopplungsfragment gebunden sind, das einen SATAund einen SMPB-Rest umfaßt, wobei der genannte SATA-Rest mit den Liposomen verbunden ist und der genannte SMPB-Rest mit dem Hormon verbunden ist.

9. Liposome nach einem der Ansprüche 1 bis 7, die mit dem Hormon durch ein Kopplungsfragment verbunden sind, das einen 4- bis 6-Kohlenstoff-Linkerarm umfaßt.

10. Liposome nach einem der Ansprüche 1 bis 9, die kleine unilamellare Vesikel sind.

11. Liposome nach einem der Ansprüche 1 bis 10, die an das Hormon über einen Aminosäurerest des Hormons gebunden sind, der nicht an der Rezeptor-Bindungsstelle lokalisiert ist und der aus Resten aus Lysin, Cystein, Arginin und Histidin ausgewählt wird.

12. Liposome nach einem der Ansprüche 1 bis 11, bei denen die Ankopplung an einem Lysin-Rest des Hormons stattfindet.

13. Methode zur Herstellung von Liposomen, die kovalent an ein Hormon gekoppelt sind, das eine biologisch aktive Zellrezeptorbindungsstelle aufweist, wobei die Methode das Freimachen der geschützten Zellrezeptorbindungsstelle des Hormons, das an Liposome nach einem der Ansprüche 1 bis 12 gekoppelt ist, umfaßt.

14. Verfahren zur Bindung von Liposomen an ein Hormon mit einer biologisch aktiven Zellrezeptorbindungsstelle, wobei die Bindung an einer Stelle des Hormons erfolgt, die nicht die genannte biologisch aktive Bindungsstelle ist, bei der die genannte Bindungsstelle vor der Ankopplung durch einen für diese Bindungsstelle spezifischen Antikörper umkehrbar geschützt wird und nach Durchführung eines beliebigen Reaktionsschrittes, der andernfalls zu einer Beeinflussung oder einem Blockieren der genannten Bindungsstelle führen könnte, freigemacht wird.

15. Verfahren nach Anspruch 14, bei dem das Freimachen nach Anbindung des Hormons an die Liposome stattfindet.

16. Verfahren nach Anspruch 14 oder Anspruch 15, bei dem der Antikörper ein monoklonaler Antikörper ist.

17. Verfahren nach einem der Ansprüche 14 bis 16, bei dem das Hormon ein zytokines Hormon ist.

18. Verfahren nach Anspruch 17, bei den das zytokine Hormon ein Interleukin 1 bis 7 ist.

19. Verfahren nach Anspruch 18, bei dem das zytokine Hormon Interleukin 2 (IL-2) ist.

20. Verfahren nach einem der Ansprüche 14 bis 19, bei dem die Liposome zumindest einen zytotoxischen Wirkstoff einschließen.

21. Verfahren nach einem der Ansprüche 14 bis 20, bei dem die Liposommembran Phosphatidylethanolamin enthält.

22. Verfahren nach einem der Ansprüche 14 bis 21, welches das Fixieren eines Kopplungsfragmentes an die Liposome umfaßt, wobei das Fragment der Bindungsrest eines Kopplungsmittels ist, das aus Succinimidyl-4-(P-Maleimidophenyl)Butyrat (SMPB) und Sulpho-Succinimidyl-Verbindungen ausgewählt ist.

23. Verfahren nach Anspruch 22, welches das Fixieren von Kopplungsfragmenten an Liposome umfaßt, wobei das Fragment ein Bindungsrest von Succinimidyl-S-Azetylthioazetat (SATA) ist.

24. Verfahren nach einem der Ansprüche 14 bis 23, welches das Fixieren eines Bindungsfragmentes an das Hormon umfaßt, wobei das Fragment der Bindungsrest eines Kopplungsmittels ist, das aus Succinimidyl-4-(P-Maleimidophenyl)Butyrat (SMPB), Sulpho-SMPB, N-(4-Carboxy-Cyclohexyl-Methyl)Maleimid (SMCC), Sulpho-SMCC, N-Maleimidobenzoyl-N-Hydroxysuccinimidester (MBS), Sulpho-MBS, N-Succinimidyl-(4-Iodoacetyl)-Aminobenzoat (SIAB) und Sulpho-SIAB ausgewählt ist.

25. Verfahren nach Anspruch 24, welches das Fixieren eines Kopplungsfragmentes, das ein Bindungsrest von Succinimidyl-4-(P-Maleimidophenyl)Butyrat (SMPB) ist, an das Hormon umfaßt.

26. Verfahren nach einem der Ansprüche 14 bis 25, welches das Fixieren eines Kopplungsfragmentes, das ein Bindungsrest von Succinimidyl-S-Azetylthioazetat (SATA) ist, an die Liposome und das Fixieren eines Kopplungsfragmentes, das ein Bindungsrest von SMPB ist, an das Hormon sowie die Reaktion der Kopplungsfragmente miteinander umfaßt.

27. Verfahren nach einem der Ansprüche 14 bis 26, bei dem die Zellrezeptorbindungsstelle des Hormons reversibel geschützt wird, bevor das Fixieren des Kopplungsfragmentes erfolgt.

28. Verfahren nach einem der Ansprüche 14 bis 27, bei dem die Liposome kleine unilamellare Vesikel sind.

29. Verfahren nach einem der Ansprüche 14 bis 28, bei dem die Liposome über einen Aminosäurerest auf dem Hormon an das Hormon gebunden werden, der nicht an der Rezeptorbindungsstelle lokalisiert ist und der aus Resten von Lysin, Cystein, Arginin und Histidin ausgewählt ist.

30. Verfahren nach Anspruch 29, bei dem die Kopplung an einen Lysin-Rest des Hormons stattfindet.

## Revendications

1. Liposomes couplés par liaison covalente à une hormone comportant un site de liaison des récepteurs cellulaires, lequel est protégé par protection réversible par un anticorps spécifique du site de liaison.

2. Liposomes selon la revendication 1, dans lesquels l'anticorps est un anticorps monoclonal.

3. Liposomes selon la revendication 1 ou la revendication 2, dans lesquels l'hormone est une hormone de type cytokine.

4. Liposomes selon la revendication 3, dans lesquels l'hormone de type cytokine est une interleukine 1 à 7.

5. Liposomes selon la revendication 4, dans lesquels l'hormone de type cytokine est l'interleukine 2 (IL-2).

6. Liposomes selon l'une quelconque des revendications 1 à 5, qui encapsulent au moins un médicament cytotoxique.

7. Liposomes selon l'une quelconque des revendications 1 à 6, dont la membrane comprend de la phosphatidyléthanolamine.

8. Liposomes selon l'une quelconque des revendications 1 à 7, qui sont couplés à l'hormone par un fragment de couplage comprenant un résidu SATA et un résidu SMPB, le résidu SATA étant lié aux liposomes et le résidu SMPB étant lié à l'hormone.

9. Liposomes selon l'une quelconque des revendications 1 à 7, qui sont couplés à l'hormone par un fragment de couplage comprenant un bras de liaison ayant de 4 à 6 atomes de carbone.

10. Liposomes selon l'une quelconque des revendications 1 à 9, qui sont de petits vésicules unilamellaires.

11. Liposomes selon l'une quelconque des revendications 1 à 10, qui sont couplés à l'hormone au niveau d'un radical aminé de l'hormone, qui n'est pas situé sur le site de liaison des récepteurs et qui est choisi parmi les radicaux lysine, cystéine, arginine et histidine.

12. Liposomes selon la revendication 11, dans lesquels le couplage a lieu au niveau d'un radical lysine de l'hormone.

13. Procédé pour préparer des liposomes couplés par liaison covalente à une hormone comportant un site de liaison biologiquement actif des récepteurs cellulaires, qui consiste à déprotéger le site de liaison protégé des récepteurs cellulaires, de l'hormone couplée aux liposomes selon l'une quelconque des revendications 1 à 12.

14. Procédé pour coupler des liposomes à une hormone comportant un site de liaison biologiquement actif des récepteurs cellulaires, le couplage s'effectuant sur un site de l'hormone qui n'est pas le site de liaison biologiquement actif, procédé dans lequel le site de liaison est protégé par une protection réversible, avant le couplage, par un anticorps spécifique du site de liaison, et est déprotégé après mise en oeuvre d'une étape de réaction quelconque qui autrement pourrait conduire à des interférences avec le site de liaison ou pourrait le bloquer.

15. Procédé selon la revendication 14, dans lequel la déprotection a lieu après liaison de l'hormone aux liposomes.

16. Procédé selon la revendication 14 ou la revendication 15, dans lequel l'anticorps est un anticorps monoclonal.

17. Procédé selon l'une quelconque des revendications 14 ou 16, dans lequel l'hormone est une hormone du type cytokine.

18. Procédé selon la revendication 17, dans lequel l'hormone du type cytokine est une interleukine 1 à 7.

19. Procédé selon la revendication 18, dans lequel l'hormone du type cytokine est l'interleukine 2 (IL-2).

20. Procédé selon l'une quelconque des revendications 14 à 19, dans lequel les liposomes encapsulent au moins un médicament cytotoxique.

21. Procédé selon l'une quelconque des revendications 14 à 20, dans lequel la membrane des liposomes comprend de la phosphatidyléthanolamine.

22. Procédé selon l'une quelconque des revendications 14 à 21, qui consiste à fixer aux liposomes un fragment de couplage, qui est le résidu de couplage d'un agent de couplage choisi parmi le 4-(p-maléimidophényl)-butyrate de succinimidyle (SMPB) et les composés succinimidylés contenant du soufre.

23. Procédé selon la revendication 22, qui consiste à fixer aux liposomes un fragment de couplage qui est le résidu de couplage du S-acétylthioacétate de succinimidyle (SATA).

24. Procédé selon l'une quelconque des revendications 14 à 23, qui consiste à fixer à l'hormone un fragment de couplage qui est le radical de couplage d'un agent de couplage choisi parmi le 4-(p-maléimidophényl)-butyrate de succinimidyle (SMPB), le sulfo-SMPB, le N-(4-carboxycyclohexylméthyl)maléimide (SMCC), le sulfo-SMCC, un ester de N-maléimidobenzoyl-N-hydroxysuccinimide (MBS), le sulfo-MBS, le 4-iodoacétylaminobenzoate de N-succinimidyle (SIAB) et le sulfo-SIAB.

25. Procédé selon la revendication 24, qui consiste à fixer à l'hormone un fragment de couplage qui est un radical de couplage du 4-(p-maléimidophényl)butyrate de succinimidyle (SMPB).

26. Procédé selon l'une quelconque des revendications 14 à 25, qui consiste à fixer à des liposomes un fragment de couplage qui est un radical de couplage du S-acétylthioacétate de succinimidyle (SATA), à coupler à l'hormone un fragment de couplage qui est un radical de couplage du SMPB, puis à faire réagir ensemble les fragments de couplage.

27. Procédé selon l'une quelconque des revendications 14 à 26, dans lequel le site de liaison des récepteurs cellulaires de l'hormone est protégé par protection réversible avant fixation du fragment de couplage.

28. Procédé selon l'une quelconque des revendications 14 à 27, dans lequel les liposomes sont de petites vésicules unilamellaires.

29. Procédé selon l'une quelconque des revendications 14 à 28, dans lequel les liposomes sont couplés à l'hormone au niveau d'un radical acide aminé se trouvant sur l'hormone, qui n'est pas situé au niveau du site de liaison des récepteurs et qui est choisi parmi les radicaux lysine, cystéine, arginine et histidine.

30. Procédé selon la revendication 29, dans lequel le couplage a lieu au niveau d'un radical lysine de l'hormone.
